# FASCICULE DE BREVET EUROPEEN

(11) **EP 3 414 335 B1**
(45) Date de publication et mention de la délivrance du brevet: **25.03.2020**
(21) Numéro de dépôt: 16710780.4
(22) Date de dépôt: 08.02.2016
(51) Int. Cl.: C12P 21/00, C12N 1/12, C12N 1/38, C12P 13/10, C12P 13/14

(54) **PROCÉDÉ D'ENRICHISSEMENT EN PROTÉINES DE LA BIOMASSE DE MICROALGUES**
VERFAHREN ZUR PROTEINANREICHERUNG VON MIKROALGENBIOMASSE
METHOD FOR THE PROTEIN ENRICHMENT OF MICROALGAL BIOMASS

(43) Date de publication de la demande: 19.12.2018
(73) Titulaire: Corbion Biotech, Inc., South San Francisco, CA 94080 (US)
(72) Inventeur: LE RUYET, Marie, 59000 Lille (FR); SEGUEILHA, Laurent, 59520 Marquette Lez Lille (FR); CAPPE, Mélanie, 59660 Merville (FR); DELAROCHE, Sylvain, 62219 Longuenesse (FR)
(74) Mandataire: Cabinet Becker et Associés
(86) Numéro de dépôt international: PCT/FR2016/050269
(87) Numéro de publication internationale: WO 2017/137668

(56) Documents cités:
- WO-A1-2015/079182
- WO-A1-2015/107312
- WO-A2-2014/154787
- SYRETT P J: "The Assimilation of Ammonia by Nitrogen-starved Cells of Chlorella vulgaris: Part II. The Assimilation of Ammonia to other Compounds", ANNALS OF BOTANY, ACADEMIC PRESS, LONDON, GB , vol. 17, no. 1 1 janvier 1953 (1953-01-01), pages 21-36, XP008177779, ISSN: 0305-7364 Extrait de l'Internet: URL:http://aob.oxfordjournals.org/content/ 17/1/21.abstract / http://www.jstor.org/stable/42908498
- SYRETT P J: "The Assimilation of Ammonia by Nitrogen-starved Cells of Chlorella vulgaris. Part I. The Correlation of Assimilation with Respiration", ANNALS OF BOTANY, ACADEMIC PRESS, LONDON, GB , vol. 17, no. 1 1 janvier 1953 (1953-01-01), pages 1-19, XP008177778, ISSN: 0305-7364 Extrait de l'Internet: URL:http://aob.oxfordjournals.org/content/ 17/1/1.full.pdf+html
- ALESSIA GUCCIONE ET AL: "Chlorella for protein and biofuels: from strain selection to outdoor cultivation in a Green Wall Panel photobioreactor", BIOTECHNOLOGY FOR BIOFUELS, BIOMED CENTRAL LTD, GB, vol. 7, no. 1, 7 juin 2014 (2014-06-07), page 84, XP021188565, ISSN: 1754-6834, DOI: 10.1186/1754-6834-7-84

## Description

La présente invention se rapporte à un procédé d'enrichissement en protéines de la biomasse de microalgues, plus particulièrement du genre *Chlorella,* plus particulièrement encore de l'espèce *Chlorella protothecoides.*

Les algues, macro et micro, ont une richesse spécifique en grande partie inexplorée. Leur exploitation à des fins alimentaire, chimique ou bioénergétique est encore très marginale. Elles recèlent cependant des composants de grande valeur, en richesse comme en abondance.

Les microalgues sont en effet des sources de vitamines, lipides, protéines, sucres, pigments et antioxydants.

Les algues et microalgues intéressent ainsi le secteur industriel qui les utilise pour la fabrication de compléments alimentaires, d'aliments fonctionnels, de cosmétiques, de médicaments ou pour l'aquaculture.

Les microalgues sont avant tout des microorganismes photosynthétiques qui colonisent tous les biotopes exposés à la lumière.

A l'échelle industrielle, leur culture monoclonale est réalisée dans des photobioréacteurs (conditions autotrophiques : à la lumière avec du CO₂) ou pour certaines d'entre elles, également dans des fermenteurs (conditions hétérotrophiques : à l'obscurité en présence d'une source carbonée).

Quelques espèces de microalgues sont en effet capables de pousser en absence de lumière : *Chlorella, Nitzschia, Cyclotella, Tetraselmis, Crypthecodinium, Schizochytrium.*

Il est d'ailleurs estimé que la culture en conditions hétérotrophiques coûte 10 fois moins chère qu'en conditions phototrophiques car, pour l'Homme du métier, ces conditions hétérotrophiques autorisent :
- l'utilisation de fermenteurs identiques à ceux utilisés pour les bactéries et les levures et permettent le contrôle de tous les paramètres de culture.
- la production de biomasses en quantité bien plus élevée que ce qui est obtenu par culture basée sur la lumière.

L'exploitation rentable des microalgues nécessite généralement la maîtrise des conditions de fermentation permettant d'accumuler ses composants d'intérêt, tels que :
- les pigments (chlorophylle a, b et c, β-carotène, astaxanthine, lutéine, phycocyanine, xanthophylles, phycoérythrine...) dont la demande est croissante, tant pour leurs remarquables propriétés antioxydantes, que pour leur apport de couleurs naturelles dans l'alimentation,
- les lipides, ceci afin d'optimiser leur contenu en acides gras (jusqu'à 60 %, voir 80 % en poids de leur matière sèche) notamment pour :
   - les applications biocarburants, mais aussi
   - les applications en Alimentation humaine ou animale, lorsque les microalgues sélectionnées produisent des acides gras polyinsaturés ou PUFAs dits "essentiels" (i.e. apportés par l'alimentation car ne sont pas naturellement produits par l'homme ou l'animal), ou
- les protéines, ceci afin d'en optimiser les qualités nutritives ou par exemple de favoriser l'apport en acides aminés d'intérêt.

Dans le cadre de l'apport en acides aminés d'intérêt, il peut être en effet avantageux de disposer de sources de protéines riches en arginine et en glutamate

L'arginine est un acide aminé qui présente de nombreuses fonctions dans le règne animal.

L'arginine peut être dégradée et ainsi servir de source d'énergie, de carbone et d'azote à la cellule qui l'assimile.

Chez divers animaux, dont les mammifères, l'arginine est décomposée en ornithine et en urée. Cette dernière est une molécule azotée qui peut être éliminée (par excrétion dans les urines) de manière à réguler la quantité de composés azotés présente dans les cellules des organismes animaux.

L'arginine permet la synthèse du monoxyde d'azote (NO) par la NO synthétase, intervenant ainsi dans la vasodilatation des artères, ce qui réduit la rigidité des vaisseaux sanguins, augmente le flux sanguin et améliore ainsi le fonctionnement des vaisseaux sanguins.

Les compléments alimentaires qui contiennent l'arginine sont recommandés pour promouvoir la santé du cœur, la fonction vasculaire, pour prévenir « l'agrégation des plaquettes » (risque de formation de caillots sanguins) et pour abaisser la pression artérielle.

L'implication de l'arginine dans la cicatrisation des plaies est liée à son rôle dans la formation de la proline, un autre acide aminé important pour la synthèse de collagène.

L'arginine est enfin un composant fréquemment utilisé, notamment par les sportifs, dans les boissons énergisantes.

L'acide glutamique quant à lui n'est pas seulement l'une des briques élémentaires utilisées pour la synthèse des protéines, mais est aussi le neurotransmetteur excitateur le plus répandu dans le système nerveux central (encéphale + moelle épinière) et est un précurseur du GABA dans les neurones GABAergiques.

Sous le code de « E620 », le glutamate est utilisé comme exhausteur de goût des aliments. Il est additionné aux préparations alimentaires pour renforcer leur goût.

Outre le glutamate, le Codex Alimentarius a reconnu aussi comme exhausteurs de goût ses sels de sodium (E621), de potassium (E622), calcium (E623), ammonium (E624) et magnésium (E625).

Le glutamate (ou ses sels) est souvent présent dans les plats tout prêts (soupes, sauces, chips, plats cuisinés). Il est aussi couramment utilisé en cuisine asiatique.

Il est actuellement fréquemment utilisé en combinaison avec des arômes dans les apéritifs (goût bacon, goût fromage). Cela permet de rehausser le goût de bacon, du fromage, etc. Il est rare de trouver un apéritif qui n'en contienne pas.

On en trouve aussi dans certaines capsules de médicaments mais pas pour ses fonctions gustatives.

Enfin, il est le composant majoritaire des auxiliaires de cuisine (bouillons cubes, fonds de sauces, sauces, etc.).

Pour parvenir à exploiter les richesses métaboliques des microalgues, de premiers procédés de fermentation permettant d'obtenir de hautes densités cellulaires (acronyme anglais : HCD pour *High-Cell-Density*) ont été ainsi beaucoup travaillés, de manière à obtenir des rendements et productivités maximales en protéines ou en lipides.

L'objectif de ces cultures HCD était l'obtention de la concentration la plus élevée possible du produit souhaité dans le laps de temps le plus court.

Ce précepte se vérifie par exemple pour la biosynthèse d'astaxanthine par *Chlorella zofingiensis,* où la croissance de la microalgue s'est avérée corrélée directement avec la production de ce composé (Wang and Peng, 2008, World J Microbiol. Biotechnol., 24(9), 1915-1922).

Cependant, le fait de maintenir la croissance à son taux maximal (µ, en h⁻¹) n'est pas toujours corrélé avec la production élevée du produit souhaité.

Il est en effet vite apparu aux spécialistes du domaine qu'il faut par exemple soumettre les microalgues à un stress nutritionnel qui limite leur croissance lorsqu'on souhaite leur faire produire d'importantes réserves lipidiques.

Il est donc procédé désormais au découplage croissance / production dans les procédés fermentaires et à la maîtrise du taux de croissance cellulaire.

En général, l'homme du métier choisit de contrôler la croissance des microalgues par la maîtrise des conditions de fermentation (Tp, pH...), ou par l'alimentation régulée en composants nutritionnels du milieu de fermentation (conditions semi continues dites « fed-batch »).

S'il choisit de contrôler la croissance des microalgues en hétérotrophie par l'apport en sources carbonées, l'homme du métier choisit généralement d'adapter la source carbonée (glucose pur, acétate, éthanol...) à la microalgue (*C. cohnii, Euglena gracilis...*) en fonction du métabolite produit (par exemple un acide gras polyinsaturé de type DHA).

La température peut-être également un paramètre clef :
- il a par exemple était rapporté que la synthèse d'acides gras polyinsaturés chez certaines espèces de microalgues, tel que l'EPA par *Chlorella minutissima,* est favorisée à une plus basse température que celle requise pour la croissance optimale de ladite microalgue;
- au contraire le rendement en lutéine est plus élevé chez *Chlorella protothecoides* cultivée en hétérotrophie, lorsque l'on augmente la température de production de 24 à 35°C.

*Chlorella protothecoides* est justement reconnue comme une des meilleures microalgues productrices d'huile.

En conditions hétérotrophiques, elle transforme rapidement les hydrates de carbone en triglycérides (plus de 50 % de sa matière sèche).

Pour optimiser cette production en triglycérides, l'homme du métier est amené à optimiser le flux carboné vers la production d'huile, en agissant sur l'environnement nutritionnel du milieu de fermentation.

Il est ainsi connu que l'accumulation en huile se produit lors d'un apport carboné suffisant, mais dans des conditions de carence en azote.

Le rapport C/N est donc ici déterminant, et il est admis que les meilleurs résultats sont obtenus en agissant directement sur la teneur en azote, la teneur en glucose n'étant pas limitante.

De manière non surprenante, cette carence en azote affecte la croissance cellulaire, ce qui résulte en un taux de croissance de 30 % plus faible que le taux de croissance normal de la microalgue (Xiong et al., Plant Physiology, 2010, 154, pp1001-1011).

Pour expliquer ce résultat, Xiong et al, dans l'article cité supra, démontrent en effet que si l'on divise la biomasse de *Chlorella* en ses 5 composants principaux, i.e. hydrates de carbone, lipides, protéines, ADN et ARN (représentant 85 % de sa matière sèche), si le rapport C/N n'a aucun impact sur la teneur en ADN, ARN et hydrates de carbone, il devient prééminent pour le contenu en protéines et en lipides.

C'est ainsi que les cellules de Chlorelles cultivées avec un rapport C/N faible contiennent 25,8 % de protéines et 25,23 % en lipides, tandis qu'un rapport C/N élevé permet la synthèse de 53,8 % de lipides et 10,5 % de protéines.

Pour optimiser sa production en huile, il est donc primordial pour l'homme du métier de contrôler le flux carboné en le déviant vers la production d'huile, au détriment de la production des protéines ; le flux carboné est redistribué et s'accumule en substances de réserve lipidiques quand les microalgues sont placées en milieu carencé en azote.

Mais *Chlorella protothecoides* peut également être avantageusement choisie pour produire des protéines.

Etant donné l'analyse faite par l'homme du métier quant à la gestion du rapport C/N pour la production d'huile (viser un rapport C/N élevé), l'homme du métier est donc amené à favoriser un rapport C/N faible, et ainsi :
- réaliser un apport important en source d'azote au milieu de fermentation, tout en maintenant constant la charge en source carbonée qui sera convertie en protéines et
- stimuler la croissance de la microalgue.

Il est ainsi choisi de modifier le flux carboné vers la production de protéines (et donc de biomasses), au détriment de la production des lipides de réserve.

La présente invention se rapporte à un procédé d'enrichissement en protéines de la biomasse de microalgues, plus particulièrement du genre *Chlorella,* plus particulièrement encore de l'espèce *Chlorella protothecoides.*

La présente invention se rapporte à un procédé d'enrichissement en protéines de la biomasse de certaines microalgues, plus particulièrement de *Chlorella protothecoides,* protéines dont la teneur en arginine et glutamine est remarquablement élevée.

La présente invention couvre plus particulièrement un procédé de production de biomasse de microalgues enrichies en protéines caractérisé en ce qu'il consiste à augmenter l'apport en ammonium dans une biomasse préalablement carencée en azote.

En effet, dans le cadre de l'invention, la société Demanderesse a au contraire choisi d'explorer une voie originale en proposant une solution alternative à celle classiquement envisagée par l'homme du métier, voire même complètement à l'opposé de celle qu'il aurait choisie.

L'invention porte ainsi sur un procédé d'enrichissement en protéines d'une microalgue cultivée en hétérotrophie, microalgue du genre *Chlorella,* plus particulièrement encore *Chlorella protothecoides,* procédé de culture hétérotrophique qui comporte :
- une première étape visant à limiter l'apport d'ammonium de manière à obtenir une biomasse de microalgues présentant une teneur en protéines inférieure à 50 % exprimée en N. 6,25, de préférence inférieure à 30 %, préférentiellement comprise entre 20 et 25 % ;
- une seconde étape dans laquelle on augmente l'apport en ammonium dans le milieu de fermentation de manière à obtenir une teneur en protéines supérieure à 50 %, de préférence supérieure à 60 %, plus préférentiellement encore supérieure à 65 %.

Comme il sera exemplifié ci-après, un mode préférentiel de réalisation du procédé conforme à l'invention peut consister à effectuer la régulation de pH dans la première étape avec un mélange NH3/KOH limitant ainsi l'apport en ammonium et ainsi favorisant la production d'une faible teneur en protéines, puis à utiliser une régulation de pH à l'NH₃ seul dans la deuxième étape, afin de réalimenter le milieu de fermentation en ammonium.

Le mélange NH3/KOH sera tel qu'il permet de limiter l'apport d'ammonium. Par exemple, pour une même concentration de NH₃ entre la première et deuxième étape, le mélange peut comprendre des rapports NH3/KOH qui sont de l'ordre d'environ de 1 : 1 comme par exemple environ 70-45 % NH₃ et 30-55 % KOH, de préférence environ 65-55 % v/v NH₃ et 35-45 % KOH, les quantités étant exprimées en mole.

Par « environ » est entendu une gamme de valeur comprenant + ou - 10 % de la valeur indiquée, de préférence + ou - 5 % de celle-ci. Par exemple, environ 10 signifie entre 9 et 11, de préférence entre 9,5 et 10,5.

Dans ce mode préférentiel de réalisation, l'apport en NH₃ est alors multiplié par environ 1,5 à 2, et la vitesse de consommation en azote qui en résulte est multipliée par 5.

Cette seconde étape est une étape durant laquelle l'augmentation de l'apport d'ammonium à une biomasse préalablement carencée en azote entraîne une surconsommation ponctuelle de ce sel par cette biomasse et conduit de manière remarquable à doper la teneur en protéines, jusqu'à une teneur supérieure à 50 %, de préférence supérieure à 60 %, préférentiellement supérieure à 65 % (% exprimés en N.6,25).

On constate ainsi que la vitesse spécifique de consommation de l'azote, qui tombe à une valeur de moins de 0,005 g/g/h pendant la phase de carence en azote augmente à une valeur de plus de 0,01 g/g/h après la levée de la carence en azote.

Dans un mode de réalisation préféré, la vitesse de croissance est maintenue substantiellement constante. Par exemple, pendant ces deux phases, le taux de croissance est maintenu à 0,07 h⁻¹ à 0,09 h⁻¹, de préférence environ 0,08 h⁻¹.

Pour exemplifier ce concept, l'invention couvre plus précisément un procédé de culture hétérotrophique de *Chlorella protothecoides* comportant :
- une phase de batch après ensemencement du fermenteur apportant 20 g/l de glucose.
- une phase de fed-batch exponentiel avec un taux de croissance fixé à 0,08 h⁻¹, déclenchée lorsque le glucose apporté en batch est totalement consommé, durant lesquelles on limite l'apport d'ammonium en utilisant une régulation de pH à l'aide d'un mélange de NH₃ et de KOH comme mentionné ci-dessus dans le but d'obtenir une biomasse présentant moins de 25 % de protéines (exprimés en N. 6,25),
   **puis**
- une phase de fed-batch exponentiel avec un même taux de croissance fixé à 0,08 h⁻¹ durant laquelle on lève la carence en ammonium en régulant le pH à l'aide d'une solution 100% ammoniaque.

Par exemple, la deuxième phase de fed-batch où on lève la carence en ammonium est initiée lorsque environ 2 kg de glucose sec ont été introduits.

Au sens de l'invention, le critère essentiel est bien que le stress cellulaire provoqué par la carence en azote du milieu de fermentation, puis une levée de ce stress, dans des conditions bien précises, déclenchent une consommation de l'azote introduit pour doper le contenu en protéines de la biomasse produite.

Cette stratégie va donc bien à l'encontre du préjugé technique selon lequel pour augmenter la teneur en protéines de la biomasse, il faut immanquablement augmenter l'apport en azote dès le début de la culture.

Par ailleurs, ces conditions opératoires se traduisent ici non seulement par une augmentation de la richesse en protéines, mais conduisent également à en augmenter de manière remarquable la teneur en arginine et en glutamate.

Plus particulièrement, comme il sera d'ailleurs exemplifié ci-après, la culture hétérotrophique des microalgues de l'espèce *Chlorella protothecoides* comprenant une étape de culture avec une carence en azote puis un pulse en ammonium conduit à produire plus de 45 % d'acide glutamique et d'arginine sur les acides aminés totaux.

Ainsi, la présente invention est également relative à un procédé d'enrichissement de la teneur en acide glutamique et/ou en arginine d'une microalgue cultivée en hétérotrophie, de préférence d'une microalgue de l'espèce *Chlorella protothecoides,* le procédé comprenant la culture hétérotrophique de ladite microalgue qui comporte une étape visant à limiter l'apport d'ammonium de manière à obtenir une biomasse de microalgues pauvre en protéines, suivi d'une étape dans laquelle on maintient le taux de croissance et augmente l'apport en ammonium.

Ces conditions de culture résultent ainsi en la préparation d'une biomasse de microalgues comprenant plus de 45 % d'acide glutamique et d'arginine sur les acides aminés totaux.

L'invention sera mieux comprise à l'aide des exemples qui suivent, lesquels se veulent illustratifs et non limitatifs.

### DESCRIPTION DES FIGURES

La figure 1 illustre l'évolution du N.6,25 en fonction du glucose consommé.
La figure 2 illustre quant à elle l'évolution de la vitesse spécifique de consommation de l'azote (qN) en fonction du glucose consommé.
Le figure 3 illustre l'évolution du N.6,25 en fonction du temps et la quantité de chaque acide aminé en % de poids de biomasse sec en fonction du temps.
Le figure 4 illustre l'évolution du N.6,25 en fonction du temps et la quantité d'acides gras totaux ou particuliers en % de poids de biomasse sec en fonction du temps.
Le figure 5 illustre l'évolution du N.6,25 en fonction du temps et la quantité de sucres totaux ou particuliers en % de poids de biomasse sec en fonction du temps.

### EXEMPLES

### Exemple 1 : Préparation d'une biomasse de C. protothecoides riche en protéines à haute teneur en acide glutamique et arginine

La souche utilisée est une *Chlorella protothecoides* (souche CCAP211/8D - *The Culture Collection of Algae and Protozoa, Scotland, UK*).

### Préculture :

- 150 mL de milieu dans un Erlenmeyer de 500 mL ;
- Composition du milieu : 40 g/L de glucose + 10 g/L d'extrait de levure. L'incubation se déroule dans les conditions suivantes :
- durée : 72 h ;
- température : 28°C ;
- agitation : 110 rpm (Incubateur Infors Multitron).

### Culture en mode batch puis fed batch

### Préparation et milieu batch initial

- préparer et filtrer un mélange KOH à 400 g/l (41 %) / NH3 à 20 % v/v (59 %) ;
- stériliser fermenteur 20 L à 121 °C/ 20 min ;
- inoculer avec 2 erlenmeyers de préculture de 500 ml (DO_{600 nm} de 15) ;
- régulation du pH à 5,2 avec le mélange KOH/NH3 ;
- agitation de 300 rpm départ ;
- aération : 15 L/min d'air ;
- régulation pO₂ à 30 % en faisant varier l'agitation;
- température : 28°C

### Alimentation

- glucose : 500 g/l
- sulfate d'ammonium : 25 g/l
- sodium phosphate monobasique : 17 g/l
- potassium phosphate monobasique : 23 g/l
- sulfate de magnésium heptahydrate : 20 g/l
- sulfate de fer : 120 mg/l
- calcium nitrate : 610 mg/l
- solution d'oligoéléments : 45 ml/l
- solution de vitamines : 3,6 ml/l

| Solution d'oligoéléments (pour 2 litres) | |
|---|---|
| Ingrédients | (g) |
| CuSO₄, 5 H₂O | 0,22 |
| ZnSO₄, 7 H₂O | 28 |
| MnSO₄, 1 H₂O | 16 |
| FeSO₄, 7 H₂O | 2,2 |
| Acide citrique | 60 |
| H₂O qsp | 2 |

| Solution de vitamines | |
|---|---|
| Ingrédients | (g/l) |
| Thiamine HCl | 13,5 |
| Biotine | 0,7 |
| Pyridoxine | 6,75 |

### Conduite de la fermentation

- apporter l'équivalent de 20 g/l de glucose avant inoculation
- quand la concentration en glucose est = 0 g/l, démarrer l'alimentation du glucose en fed-batch ; utiliser un débit permettant de fixer le taux de croissance à 0.08 h-1.
- régulation du pH à 5,2 avec le mélange 41 % KOH / 59 % NH₃
- quand 2 kg de glucose sont consommés par la microalgue, on passe en régulation du pH par NH₃ seul,
- quand la biomasse atteint 100 g/l en poids de matière sèche, et qu'environ 3,5 kg de glucose ont été envoyés, l'alimentation en glucose est stoppée.

### Résultats :

Deux essais ont été réalisés dans ces mêmes conditions et les résultats sont présentés dans le tableau I et les graphes suivants :

**Tableau I.**

| | Essai 1 F2 140519 | Essai 2 F5 140623 |
|---|---|---|
| | Titre final (%) (pour 3,6 kg de glucose consommé) | Titre final (%) (pour 3,4 kg de glucose consommé) |
| N.6,25 | 66,0 | 65,7 |
| Acides aminés totaux | 44,7 | 43,2 |
| Teneur en Arg et Glu sur acides aminés totaux | 46 | 47 |
| Acides gras totaux | 10,2 | 10,1 |
| Sucres totaux | 20,3 | 21,7 |
| Coloration de la biomasse | Jaune | Jaune |

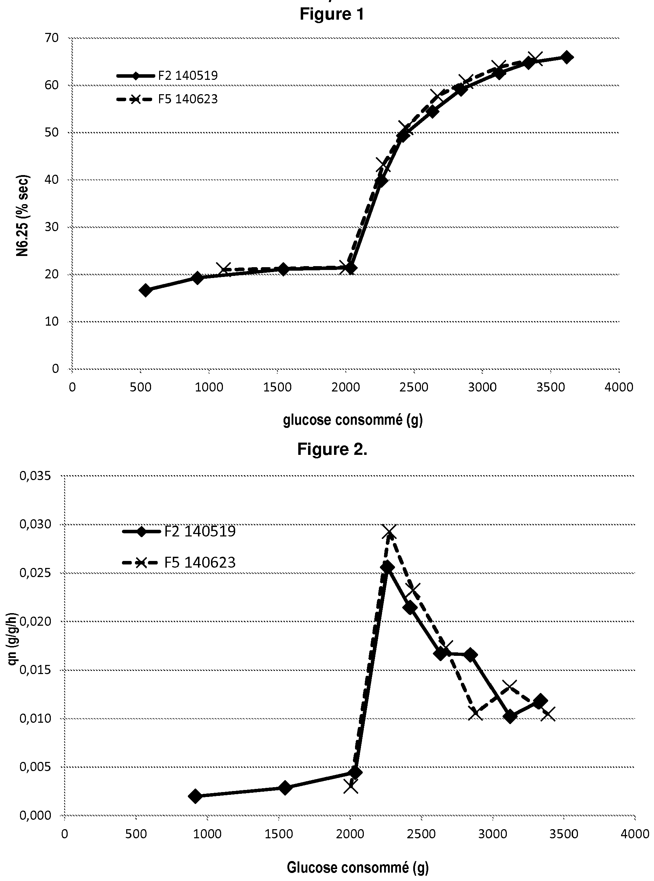

La figure 1 illustre l'évolution du N.6,25 en fonction du glucose consommé. Ces deux essais traduisent de remarquables résultats : l'obtention d'une biomasse jaune avec un taux de N6,25 de plus de 65 %.

La figure 2 illustre quant à elle l'évolution de la vitesse spécifique de consommation de l'azote (qN) en fonction du glucose consommé.

Il apparait que la vitesse spécifique de consommation d'azote est à son maximum après la levée de la limitation en azote (à 2 kg de glucose consommé), puis diminue progressivement. Les vitesses similaires entre les 2 essais traduisent également la bonne répétabilité du protocole.

Une analyse complète des acides aminés présents dans la biomasse a été réalisée sur un échantillon prélevé juste avant la levée de la limitation et sur plusieurs échantillons après le pulse.

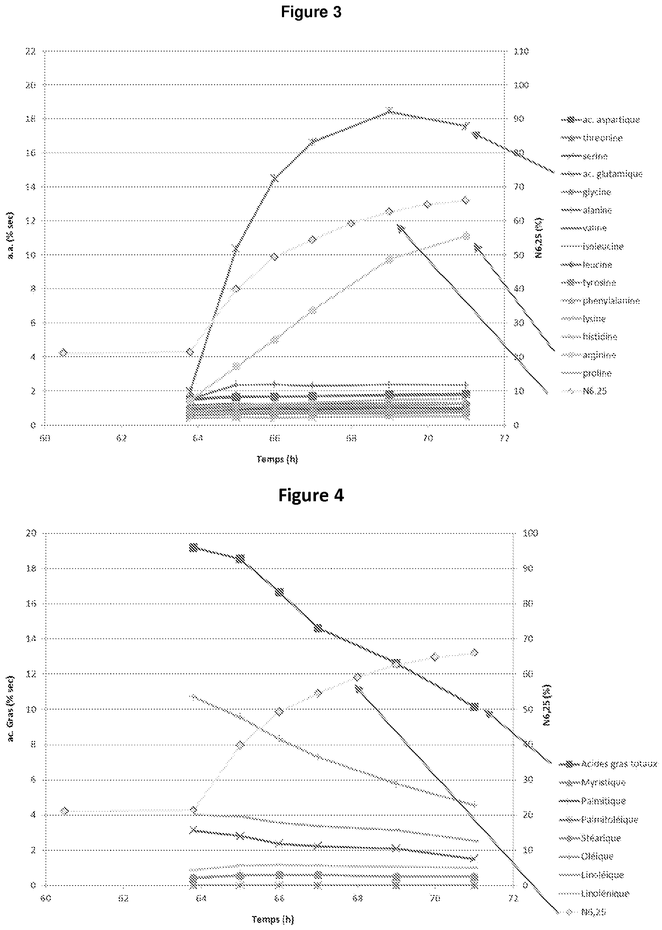

Les résultats sont représentés dans la figure 3.

On note que juste avant la levée de la limitation en azote, la somme des acides aminés est faible (16,3%), et il n'y a pas de prédominance parmi les différents acides aminés.

Une heure après la levée de la limitation en azote, on remarque que l'acide aminé qui connait la remontée la plus forte est l'acide glutamique, suivi de l'arginine. Le taux des autres acides aminés augmente également mais de manière beaucoup plus faible.

L'augmentation du N6.25 est donc surtout corrélée avec l'augmentation de l'acide glutamique et de l'arginine.

En complément de ces analyses, une analyse complète des acides gras présents dans la biomasse a été réalisée sur un échantillon prélevé juste avant la levée de la limitation en azote et sur plusieurs échantillons après le pulse.

Les résultats sont représentés dans la figure 4.

Le taux d'acides gras totaux dans la biomasse qui est de 19.2% avant le pulse baisse jusqu'à 10.2%. L'acide gras majoritaire qui suit cette courbe est l'acide oléique.

Les acides gras s'accumulent donc dans la biomasse lorsque celle-ci est carencée en azote.

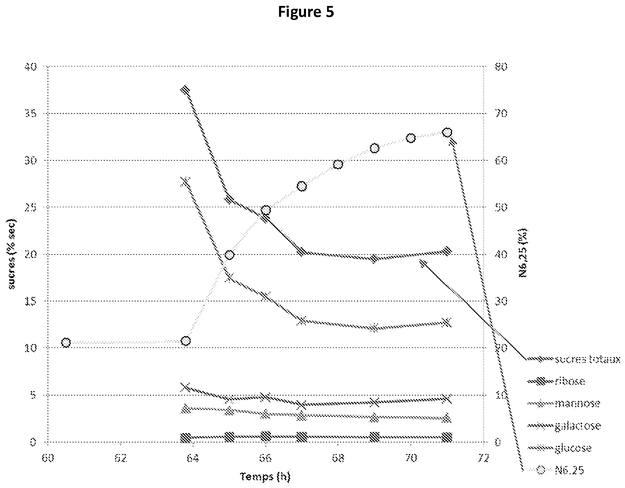

Une analyse complète des sucres présents dans la biomasse a été également réalisée sur un échantillon prélevé juste avant la levée de la limitation en azote et sur plusieurs échantillons après la levée de la limitation en azote. Les résultats sont représentés dans la figure 5.

Le taux de sucres totaux dans la biomasse qui est de 37.5% avant la levée de la limitation en azote baisse jusqu'à 20% puis stagne. Le sucre majoritaire qui suit cette courbe est le glucose.

Les sucres sont donc également stockés dans la biomasse lorsque celle-ci est carencée en azote.

Le taux de sucres semble se stabiliser ensuite, contrairement au taux d'acide gras qui ne fait que diminuer.

La charge en sels de la biomasse a été mesurée par la mesure du résidu à la calcination : il est ici de 9 %.

## Revendications

1. Procédé d'enrichissement en protéines d'une microalgue cultivée en hétérotrophie, microalgue du genre *Chlorella,* plus particulièrement encore *Chlorella protothecoides,* **caractérisé en ce qu'**il comporte :
- une première étape visant à limiter l'apport d'ammonium de manière à obtenir une biomasse de microalgues présentant une teneur en protéines, inférieure à 50 % exprimée en N. 6,25, de préférence inférieure à 30 %, plus préférentiellement comprise entre 20 et 25 % ;
- une seconde étape dans laquelle on augmente l'apport en ammonium dans le milieu de fermentation de manière à obtenir une teneur en protéines supérieure à 50 %, de préférence supérieure à 60 %, préférentiellement encore supérieure à 65 %.

2. Procédé selon la revendication 1, **caractérisé en ce que** la régulation de pH est réalisée dans la première étape par un mélange NH3/KOH, et est réalisée dans la seconde par du NH₃ seul.

3. Procédé selon l'une ou l'autre des revendications 1 et 2, **caractérisé en ce que** le mélange NH₃/KOH est d'environ 70-45 % NH₃ et 30-55 % KOH, de préférence environ 65-55 % v/v NH₃ et 35-45 % KOH, les quantités étant exprimées en mole.

4. Procédé selon l'une quelconque des revendications 1 à 3, **caractérise en ce que** l'apport en ammonium est multiplié par environ 1,5 à 2.

5. Procédé selon l'une quelconque des revendications 1 à 4, **caractérisé en ce que** dans la première étape, la vitesse spécifique de consommation de l'azote par la microalgue est inférieure à 0,005 g/g/h et que dans la seconde étape, elle est supérieure à 0,01 g/g/h.

6. Procédé selon l'une quelconque des revendications 1 à 5, **caractérisé en ce que** le taux de croissance est maintenu substantiellement constant pendant la première et deuxième phase, de préférence maintenu à 0,07 h⁻¹ à 0,09 h⁻¹, de préférence environ 0,08 h⁻¹.

7. Procédé selon l'une quelconque des revendications 1 à 6, caractérise én ce qu'il comporte :
- une phase de batch, après ensemencement du fermenteur, apportant 20 g/l de glucose,
- une phase de fed-batch exponentiel avec un taux de croissance fixé à 0,08 h¹, déclenchée lorsque le glucose apporté en batch est totalement consommé, durant lesquelles on limite l'apport d'ammonium en utilisant une régulation de pH à l'aide d'un mélange de NH₃ et de KOH dans le but d'obtenir une biomasse présentant moins de 25 % de protéines (exprimés en N. 6,25),
**puis**
- une phase de fed-batch exponentiel avec un même taux de croissance fixé à 0,08 h⁻¹ durant laquelle on lève la limitation en ammonium en régulant le pH à l'aide d'une solution 100% ammoniaque.

8. Procédé selon l'une quelconque des revendications 1 à 7, **caractérisé en ce qu'**il conduit à produire une biomasse de microalgues présentant plus de 45 % d'acide glutamique et d'arginine sur acides aminés totaux composant ladite biomasse.

## Patentansprüche

1. Verfahren zur Anreicherung von Proteinen einer heterotroph kultivierten Mikroalge der Gattung *Chlorella,* insbesondere *Chlorella protothecoides,* **dadurch gekennzeichnet, dass** es umfasst:
- einen ersten Schritt der Limitierung der Zufuhr von Ammonium, um eine Mikroalgenbiomasse zu erhalten, die einen Proteingehalt unter 50%, ausgedrückt als N 6,25, bevorzugt unter 30%, bevorzugter zwischen 20 und 25% aufweist;
- einen zweiten Schritt, in dem die Zufuhr von Ammonium in das Fermentationsmedium erhöht wird, um einen Proteingehalt über 50%, bevorzugt über 60%, noch bevorzugter über 65% zu erhalten.

2. Verfahren gemäß Anspruch 1, **dadurch gekennzeichnet, dass** die pH-Regelung im ersten Schritt mithilfe eines NH₃/KOH-Gemisches erfolgt und im zweiten Schritt ausschließlich mit NH₃ erfolgt.

3. Verfahren gemäß einem der Ansprüche 1 und 2, **dadurch gekennzeichnet, dass** das NH₃/KOH-Gemisch ungefähr 70-45% NH₃ und 30-55% KOH, bevorzugt ungefähr 65-55% v/v NH₃ und 35-45% KOH enthält, wobei die Mengen in Mol ausgedrückt sind.

4. Verfahren gemäß einem der Ansprüche 1 bis 3, **dadurch gekennzeichnet, dass** die Zufuhr von Ammonium auf ungefähr das 1,5- bis 2fache erhöht wird.

5. Verfahren gemäß einem der Ansprüche 1 bis 4, **dadurch gekennzeichnet, dass** im ersten Schritt die spezifische Verbrauchsrate von Stickstoff von der Mikroalge unter 0,005 g/g/h liegt und im zweiten Schritt die spezifische Verbrauchsrate über 0,01 g/g/h liegt.

6. Verfahren gemäß einem der Ansprüche 1 bis 5, **dadurch gekennzeichnet, dass** die Wachstumsrate während der ersten und zweiten Phase im Wesentlichen konstant gehalten wird, bevorzugt auf 0,07 h⁻¹ bis 0,09 h⁻¹, bevorzugt auf ungefähr 0,08 h⁻¹ gehalten wird.

7. Verfahren gemäß einem der Ansprüche 1 bis 6, **dadurch gekennzeichnet, dass** es umfasst:
- eine Batch-Phase, wobei nach Animpfen des Fermenters 20 g/l Glucose zugeführt wird,
- eine exponentielle Fed-Batch-Phase mit einer auf 0,08 h⁻¹ gehaltenen Wachstumsrate, wobei diese Phase ausgelöst wird, wenn die in der Batch-Phase zugeführte Glucose vollständig verbraucht ist,
während dieser Phasen die Zufuhr von Ammonium unter Verwendung einer pH-Regelung mithilfe eines Gemisches von NH₃ und KOH mit dem Ziel limitiert ist, eine Biomasse zu erhalten, die weniger als 25% Proteine aufweist (ausgedrückt als N 6,25),
dann
- eine exponentielle Fed-Batch-Phase mit einer gleichen auf 0,08 h⁻¹ gehaltenen Wachstumsrate, während dieser Phase die Limitierung von Ammonium durch Regelung des pH mithilfe einer 100% Ammoniaklösung aufgehoben wird.

8. Verfahren gemäß einem der Ansprüche 1 bis 7, **dadurch gekennzeichnet, dass** es zu einer Produktion einer Mikroalgenbiomasse führt, die mehr als 45% Glutaminsäure und Arginin bezogen auf die gesamten in besagter Biomasse enthaltenen Aminosäuren aufweist.

## Claims

1. A method for the protein enrichment of a heterotrophically cultured microalga, the microalga being of the genus *Chlorella,* even more particularly *Chlorella protothecoides,* **characterized in that** it comprises:
- a first step directed toward limiting the ammonium supply so as to obtain a microalgal biomass with a protein content of less than 50% expressed as N.6.25, preferably less than 30%, more preferentially between 20 and 25%;
- a second step in which the ammonium supply in the fermentation medium is increased so as to obtain a protein content of greater than 50%, preferably greater than 60%, more preferentially greater than 65%.

2. The method as claimed in claim 1, **characterized in that** the pH regulation is performed in the first step with an NH₃/KOH mixture, and is performed in the second with NH₃ alone.

3. The method as claimed in either of claims 1 and 2, **characterized in that** the NH₃/KOH mixture is about 70-45% NH₃ and 30-55% KOH, preferably about 65-55% v/v NH₃ and 35-45% KOH, the amounts being expressed in moles.

4. The method as claimed in any one of claims 1 to 3, **characterized in that** the ammonium supply is multiplied by about 1.5 to 2.

5. The method as claimed in any one of claims 1 to 4, **characterized in that**, in the first step, the specific rate of nitrogen consumption by the microalga is less than 0.005 g/g/h, and that, in the second step, it is greater than 0.01 g/g/h.

6. The method as claimed in any one of claims 1 to 5, **characterized in that** the growth rate is kept substantially constant during the first and second phase, preferably maintained at 0.07 h⁻¹ to 0.09 h⁻¹, preferably about 0.08 h⁻¹.

7. The method as claimed in any one of claims 1 to 6, **characterized in that** it comprises:
- a batch phase, after seeding the fermenter, supplying 20 g/l of glucose,
- an exponential fed-batch phase with a growth rate set at 0.08 h⁻¹, started when the glucose supplied in the batch is totally consumed,
during which the ammonium supply is limited by using pH regulation with the aid of a mixture of NH₃ and of KOH for the purpose of obtaining a biomass containing less than 25% protein (expressed as N.6.25),
and then
- an exponential fed-batch phase with the same growth rate set at 0.08 h⁻¹, during which the ammonium limitation is lifted by regulating the pH with the aid of a 100% aqueous ammonia solution.

8. The method as claimed in any one of claims 1 to 7, **characterized in that** it leads to producing a microalgal biomass containing more than 45% glutamic acid and arginine relative to the total amino acids of which said biomass is composed.
